# EUROPEAN PATENT APPLICATION

(11) **EP 4 349 317 A1**
(43) Date of publication of application: **10.04.2024**
(21) Application number: 22200417.8
(22) Date of filing: 07.10.2022
(51) Int. Cl.: A61K 8/02, A61K 8/04, A61K 8/27, A61K 8/29, A61K 8/37, A61Q 17/04

(54) **STABLE DISPERSIONS OF TITANIUM DIOXIDE AND/OR ZINC OXIDE**

(71) Applicant: Oleon N.V., 9940 Ertvelde (BE)
(72) Inventor: MOGARAJA, Sanggari, 47130 Puchong, Selangor (MY); VAN DER WEEËN, Pieter, 9040 SINT-AMANDSBERG (Gent) (BE)
(74) Representative: Santarelli

(57) **Abstract**

The present invention relates to the use of diisoamyl malate as a dispersing agent of titanium dioxide and zinc oxide, combination and compositions comprising them, and their uses as cosmetic products.

## Description

The present invention relates to the use of a specific emollient as a dispersing agent of titanium dioxide and/or zinc oxide.

Titanium dioxide and zinc oxide are compounds that can be used as inorganic or mineral ultraviolet (UV) filters. Those inorganic UV filters are considered safer than organic UV filters for the consumer and the environment and are accepted in sunscreens labelled as organics.

An inconvenient of those inorganic UV filters is the whitening effect they may have. Indeed, due to the white color of titanium dioxide and zinc oxide powders, white traces can remained when applied on the skin.

To limit this whitening effect, inorganic UV filters are usually used with an average particle size smaller than 100 nm.

Although the whitening effect is reduced and the dispersion of those nanoparticles is easier, there are safety concerns surrounding those nanoparticles, in particular about skin penetration, risk of inhalation, eco-toxicity and bioaccumulation in the human body.

Also, there is a need for an efficient dispersing agent of titanium dioxide and zinc oxide, in particular for those having an average particle size greater than 100 nm, that would advantageously combine the following technical effects:
- a good spreading of titanium dioxide and zinc oxide on the skin for a better protection of the skin;
- a skin protection factor (SPF) boosting effect of titanium dioxide and/or zinc oxide
- a minimal residual whitening effect on the skin.

Indeed, the better the titanium dioxide and/or zinc oxide is/are well dispersed, the better they are uniformly spread on the skin and the better the protection of the skin.

The SPF boosting effect is whereby a dispersing agent is able to retain or enhance the SPF value given by the UV Filter(s).

Furthermore, if the dispersing agent does not affect negatively the SPF of titanium dioxide and/or zinc oxide, there is no need to add additional quantities of UV filters to reach a specific SPF.

In the cosmetic field, some emollients, such as C12-15 alkyl benzoate, can be used to solubilize some solid organic UV filters. However, it is more difficult for those emollients to disperse solid inorganic UV filters.

The Applicant surprisingly found that a specific emollient could so efficiently disperse some inorganic UV filters, that resulting compositions could be spread with acceptable whitening effect and their SPF were not reduced.

Accordingly, the present invention relates to the use of diisoamyl malate as a dispersing agent of titanium dioxide and/or zinc oxide.

Diisoamyl malate is indeed a particularly good dispersing agent for both titanium dioxide and zinc oxide, since the respective dispersions obtained are stable.

By dispersing agent, also named a dispersant, it is intended to mean a liquid chemical which is able to keep particles of another chemical in suspension by preventing their aggregation.

By stable, it is intended to mean that at 25°C at atmospheric pressure, the dispersion remains homogeneous for at least 24 hours after the preparation of the dispersion.

Advantageously, in the use according to the invention, the titanium dioxide and/or zinc oxide present(s) a particle size larger than 100 nm.

By particle size it is intended to mean the average particle size which is the average diameter based on the median particle sizes distribution of all particles representing 50% of the particle volume.

Diisoamyl malate is not only a good dispersing agent for nanoparticles, but also for larger particles.

Examples 1 and 2 illustrate this dispersing property, where 20% by weight of respectively titanium dioxide and zinc oxide with particle sizes larger than 100 nm, could be homogeneously dispersed in 80% by weight of diisoamyl malate. Both dispersions remained homogeneous at least 24 hours at 25°C.

Preferably, the particle size of titanium dioxide and/or zinc oxide is of at least 120 nm, more preferably of at least 130 nm.

The titanium dioxide and/or zinc oxide may be coated or not. The coatings might be silica (SiO2), aluminum hydroxide (AI2(OH)3), aluminum oxide (Al2O3), alumina, sodium hexametaphosphate (Na(PO3)6), sodium metaphosphate (Na(PO3)n), aluminum stearate, stearic acid, lauric acid, dimethylpolysiloxane, dimethicone, methylypolysiloxane, simethicone, either as single components or as mixtures.

Process for obtaining a stable dispersion of titanium dioxide and/or zinc oxide, comprising a step of stirring the titanium dioxide and/or zinc oxide in diisoamyl malate.

Preferably the stirring is of at least 600 rpm, more preferably of at least 700 rpm, even more preferably of at least 800 rpm, such as 1000 rpm.

The present invention relates to a combination consisting of diisoamyl malate and titanium dioxide and/or zinc oxide.

The titanium dioxide and/or zinc oxide is as described above, including preferential and advantageous features.

Preferably, the weight ratio quantity of titanium dioxide and zinc oxide / quantity of diisoamyl malate, is comprised between 0.1 and 2.5, more preferably between 0.2 and 2.

Preferably, the combination is in the form of a homogeneous dispersion of titanium dioxide and/or zinc oxide in diisoamyl malate. This dispersion is stable, i.e. it remains as such for at least 24 hours at 25°C and atmospheric pressure.

In the present application, unless otherwise indicated, all ranges of values used are to be understood as being inclusive limits.

The present invention also relates to a process for preparing a combination according to the invention, by mixing diisoamyl malate with titanium dioxide and/or zinc oxide.

To obtain an homogeneous dispersion, the mixing is preferably performed with a mechanical stirrer at a stirring rate of at least 600 rpm, more preferably of at least 700 rpm, even more preferably of at least 800 rpm, such as 1000 rpm.

During the mixing, it is not necessary to heat. The mixing may be performed at room temperature, such as at a temperature of 25°C +/- 5°C.

The present invention also relates to a composition comprising:
- titanium dioxide and/or zinc oxide having a particle size larger than 100 nm; and
- diisoamyl malate.

Preferably, the particle size of titanium dioxide and zinc oxide is of at least 120 nm, more preferably of at least 130 nm.

Preferably, the quantity of titanium dioxide and/or zinc oxide having a particle size larger than 100 nm, is of at least 5%, more preferably of at least 8%, even more preferably of at least 10% by weight based on the weight of the composition.

Preferably, the quantity of titanium dioxide and/or zinc oxide having a particle size larger than 100 nm, is of at most 50%, more preferably of at most 40%, even more preferably of at most 30% by weight, such as of at most 25% by weight based on the weight of the composition.

Preferably, the quantity of titanium dioxide and/or zinc oxide having a particle size larger than 100 nm, is comprised between 5 and 50%, such as between 5 and 40%, 5 and 30%, more preferably between 10 and 30%, such as 10 and 25% by weight based on the weight of the composition.

Advantageously, in the composition according to the invention, the quantity of diisoamyl malate is of at least 8% by weight based on the weight of the composition.

Preferably, the quantity of diisoamyl malate is of least 9%, more preferably of at least 10% by weight based on the weight of the composition.

Preferably, the quantity of diisoamyl malate is of at most 50%, more preferably of at most 25%, even more preferably of at most 20% by weight based on the weight of the composition.

Advantageously, the composition according to the invention, further comprises a thickening and/or gelling agent.

The thickening and/or gelling agent may be a single compound having both properties. Alternatively, the composition can comprise a thickening agent, or a gelling agent, or a thickening agent and a gelling agent.

Preferably, the total quantity of thickening and/or gelling agent is of at least 0.1%, more preferably of at least 0.15%, even more preferably of at least 0.2% by weight based on the weight of the composition.

Preferably, the total quantity of thickening and/or gelling agent is of at most 65%, more preferably of at most 60% by weight based on the weight of the composition.

By total quantity of thickening and/or gelling agent, it is intended to mean the quantity of all thickening and/or gelling agent(s) present in the composition.

The thickening and/or gelling agent may be chosen among the group consisting of mixture of isohexatriocontentol, dilinoleyl alcohol, glycerol monocaprylate and ethyl cellulose; dibutyl ethylhexanoyl glutamide; dibutyl lauroyl glutamide; gums such as xanthan, carrageenan; polysaccharides such as cellulose and starch; cocamide DEA; polymers of acrylic acid such as carbomer; and mixtures thereof.

A preferred composition according to the invention comprises:
- 5-50% by weight of titanium dioxide and/or zinc oxide having a particle size larger than 100 nm;
- 8-25% by weight of diisoamyl malate; and
- 0.1-65% by weight of a thickening and/or gelling agent.

A particularly preferred composition according to the invention comprises:
- 10-30% by weight of titanium dioxide and/or zinc oxide having a particle size larger than 100 nm;
- 10-20% by weight of diisoamyl malate; and
- 0.2-60% by weight of a thickening and/or gelling agent.

The composition according to the invention may further comprise a preservative, an emulsifier, a solid organic UV filter, a liquid organic UV filter, a solvent, an active ingredient and/or a perfume.

More particularly, the preservative is chosen among the group consisting of phenoxyethanol, glycol ethers, benzoic acid, sorbic acid, salicylic acid, glycols, parabens, thiazolinones, ethylhexylglycerin, aldehydes; and mixtures thereof.

More particularly, the active ingredient is chosen among the group consisting of allantoin, tocopherol, ascorbic acid, ascorbyl palmitate, vitamin D, polyphenols, flavonoids, and mixtures thereof.

More particularly, the solid organic UV filter is chosen among the group consisting of butyl methoxydibenzoylmethane, benzophenone-3, diethylamino hydroxybenzoyl hexyl benzoate, bis-ethylhexyloxyphenol methoxyphenyl triazine, ethylhexyl triazone, and mixtures thereof. Preferably, the solid organic UV filter is chosen among the group consisting of butyl methoxydibenzoylmethane, benzophenone-3, diethylamino hydroxybenzoyl hexyl benzoate, ethylhexyl triazone, and mixtures thereof.

More particularly, the liquid organic UV filter is chosen among the group consisting of ethylhexyl methoxycinnamate, octocrylene, and mixtures thereof.

More particularly, the solvent is chosen among the group consisting of water, ethanol, glycerin, and mixtures thereof.

The emulsifier can be any emulsifier known by the skilled person.

Preferably, the composition according to the invention is free of organic UV filter. The composition according to the invention is then a mineral based composition containing exclusively inorganic UV filter(s) as UV filters.

In a first embodiment, the composition according to the invention is free of water.

Advantageously, the composition according to the invention is free of water.

The composition according to the invention is then an oily composition.

If a thickening and/or gelling agent is present, then it is an oil soluble agent.

The composition according to the first embodiment may be in the form of a gel or a stick, i.e. a hard gel, as illustrated in Example 3.2.

Preferably, the total quantity of thickening and/or gelling agent is of at least 20%, more preferably of at least 30% by weight based on the weight of the composition.

The thickening and/or gelling agent may be chosen among the group consisting of mixture of isohexatriocontentol, dilinoleyl alcohol, glycerol monocaprylate and ethyl cellulose; dibutyl ethylhexanoyl glutamide; dibutyl lauroyl glutamide; and mixtures thereof.

In a second embodiment, the composition according to the invention, further comprises water.

Advantageously, the composition according to the invention is in the form of an oil-in-water emulsion.

Preferably, the quantity of water is of at least 50% by weight, more preferably of at least 60% by weight based on the weight of the composition.

Preferably, the total quantity of thickening and/or gelling agent is of at most 1%, more preferably of at most 0.5%, even more preferably of at most 0.4% such as of at most 0.3% by weight based on the weight of the composition.

Preferably, the total quantity of the thickening and/or gelling agent is comprised between 0.1 and 1 %, such as between 0.1 and 0.5%, 0.1 and 0.4%, 0.1 and 0.3% by weight, more preferably between 0.15 and 0.4%, such as 0.15 and 0.3% by weight, even more preferably between 0.2 and 0.4%, such as 0.2 and 0.3% by weight based on the weight of the oil-in-water emulsion.

The thickening and/or gelling agent may be chosen among the group consisting of gums such as xanthan, carrageenan; polysaccharides such as cellulose and starch; cocamide DEA; polymers of acrylic acid such as carbomer; and mixtures thereof.

The composition according to the invention may be in the form of a gel cream, a liquid or a gel. Example 3.1 describes a composition in the form of a liquid, such as a spray.

The compositions according to the invention may exhibit high SPF. This is due to the presence of diisoamyl malate, which also acts as a SPF booster. It can be seen in Example 3.3, that the SPF of a composition according to the invention is similar or even higher than the estimated SPF of same type and quantity of inorganic UV filters alone.

Moreover, the compositions according to the invention, even in presence of titanium dioxide and/or zinc oxide having a particle size larger than 100 nm more difficult to dispersed, can be spread on a surface without leaving a significant white trace.

In Example 3.4, it is shown that the whitening effect is very limited with composition according to the invention comprising diisoamyl malate instead of another dispersing agent.

Diisoamyl malate allows homogeneous dispersion of both titanium dioxide and zinc oxide, even when their particle sizes are larger than 100 nm, without affecting their function of ultraviolet filters and with limited whitening effect.

The present invention also concerns a process of preparation of a composition according to the invention, comprising a step of dispersing titanium dioxide and/or zinc oxide in diisoamyl malate.

The titanium dioxide and/or zinc oxide and diisoamyl malate are as described above, including preferential and advantageous features.

Preferably, the process according to the invention, further comprise a step of mixing a thickening and/or gelling agent with diisoamyl malate.

The thickening and/or gelling agent is as described above, including preferential and advantageous features.

In a first embodiment of the process, the resulting composition is free of water.

Titanium dioxide and/or zinc oxide is/are first dispersed in diisoamyl malate to form a combination according to the invention.

A thickening and/or gelling agent may be mixed directly to diisoamyl malate, or first solubilized in an emollient other than diisoamyl malate.

Preferably, the process of preparation is conducted at a temperature of at most 90°C, more preferably at a temperature comprised between 60 and 90°C, even more preferably between 75 and 85°C.

In a second embodiment of the process, the resulting composition is an oil-in-water emulsion.

The process according to the invention, then further comprises a step of stirring an oily phase comprising diisoamyl malate into an aqueous phase.

The titanium dioxide and/or zinc oxide may be dispersed in diisoamyl malate, in the oily phase prior and/or after the emulsification step.

A thickening and/or gelling agent may be mixed to diisoamyl malate, preferably it is first solubilized in the aqueous phase.

An emulsifier can be added into the oily phase, prior to the addition into the aqueous phase.

Preferably, the oily phase and the aqueous phases are prepared at a temperature of at most 90°C, more preferably at a temperature comprised between 60 and 90°C, even more preferably between 75 and 85°C.

The present invention also concerns the use of the composition according to the invention, as a cosmetic product.

The cosmetic product can be in the form of a gel, a gel cream, a stick, a spray or a lotion.

The cosmetic product can be a waterless formulation or not.

The cosmetic product can be a skin product, a hair product or a product for make-up such as a lipstick.

More particularly, the cosmetic product may be a mineral sunscreen product.

The cosmetic product can further comprise additional ingredients that are typically used in cosmetics, such as, an active ingredient, a preservative, and/or a perfume.

The present invention is further described in the following examples, given by way of illustration, with reference to the figures:
- Figure 1 represents a diagram relating to the evolution of the viscosity of combination C1 according to the shear rate applied, the combination C1 consisting of 20% by weight of titanium dioxide in 80% by weight of diisoamyl malate;
- Figure 2 represents a diagram relating to the evolution of the viscosity of combination C2 according to the shear rate applied, the combination C2 consisting of 20% by weight of zinc oxide in 80% by weight of diisoamyl malate.

### Chemicals used in Examples

- Inorganic UV filters:
   - titanium dioxide : Kronos 1171 (average particle size: 136.8-140.8 nm) from Kronos;
   - zinc oxide: Zinc Oxide pharma grad (average particle size: 70-200 nm), from Approfit;
- Emollients:
   - diisoamyl malate: prepared by esterification of malic acid with isoamyl alcohol;
   - C12-15 alkyl benzoate : Finsolv TN from Innospec;
   - propylene glycol diheptanoate: Jolee 7202 from Oleon;
   - sunflower (Helianthus) seed oil: Radia 6122 from Oleon;
- Emulsifier: glyceryl stearate and PEG-100 stearate: Radia 7490 from Oleon;
- Stick stabilizer: polyglyceryl-3 diisostearate: Jolee 7245 from Oleon;
- Thickenings and/or gelling agents:
   - carbomer: Carbopol Ultrex 10 from Lubrizol;
   - isohexatriocontentol, dilinoleyl alcohol, glycerol monocaprylate, ethyl cellulose : Jolee 7799 from Oleon;
   - dibutyl ethylhexanoyl glutamide (and) dibutyl lauroyl glutamide (and) octyldodecanol: AJK-OD2046 from Ajinomoto;
- Tocopherol acetate: vitamin E from BASF;
- Phenoxyethanol from Arkema;
- Allantoin: Ronacare Allantoin from Merck.

### Example 1: Preparation of combinations

### 1.1 Combinations C1-C2 according to the invention

Combinations C1 and C2 were prepared by adding respectively 2 g of titanium dioxide or 2 g of zinc oxide into 8 g of diisoamyl malate under stirring at 1000 rpm.

### 1.2 Comparative combinations

Comparative combinations CC11 and CC21 were prepared by adding respectively 2 g of titanium dioxide or 2 g of zinc oxide in 8 g of C12-15 alkyl benzoate under stirring at 1000 rpm.

Comparative combinations CC12 and CC22 were prepared by adding respectively 2 g of titanium dioxide or 2 g of zinc oxide in 8 g of propylene glycol diheptanoate under stirring at 1000 rpm.

Combinations thus prepared were in the form of homogeneous dispersions of inorganic UV filters.

### Example 2: Characteristics of the combinations

### 2.1 Stability

The combinations prepared in Example 1 were maintained 24 hours at 25°C.

After this period, the combinations C1 and C2 were still homogeneous dispersions of their respective inorganic UV filter into diisoamyl malate, as comparative combinations CC12 and CC22.

On the opposite, comparative combinations CC11 and CC21 were no more homogeneous dispersions, but bi-phasic systems with a solid phase (UV filter) and a liquid phase (C12-15 alkyl benzoate).

### 2.2 Wetting performance

To evaluate the wetting performance of the combinations, contact angles of combinations and of three blank samples (a first one with diisoamyl malate, a second one with C12-15 alkyl benzoate and a third one with propylene glycol diheptanoate solely) were measured according to the method described in the standard ASTM D7334-08, using the DSA100 Drop Shape Analyzer (Kruss scientific).

The impact of the diisoamyl malate, C12-15 alkyl benzoate and propylene glycol diheptanoate, on the contact angle values of combinations is determined by calculating the variation of values between the value of contact angle of a combination and the value of contact angle of the corresponding blank (with similar dispersant).

Results are gathered in Table 1 below.

**Table 1: Variations of contact angles of combinations C1 and C2 according to the invention and of comparative combinations CC11, CC12, CC21 and CC22.**

| | Dispersing agent | Inorganic UV filter | Variation of contact angles (%) |
|---|---|---|---|
| C1 | diisoamyl malate | titanium dioxide | -72.1 |
| CC11 | C2-15 alkyl benzoate | titanium dioxide | +7.6 |
| CC12 | propylene glycol diheptanoate | titanium dioxide | -13.4 |
| C2 | diisoamyl malate | zinc oxide | -82.5 |
| CC21 | C2-15 alkyl benzoate | zinc oxide | -13.1 |
| CC22 | propylene glycol diheptanoate | zinc oxide | -13.4 |

The contact angles of combinations according to the invention are reduced for both inorganic UV filters, titanium dioxide and zinc oxide, dispersed in diisoamyl malate (-72.1% for C1 and -82.5% for C2).

Moreover, it can be observed that the variations of contact angles are, by far, the most negatives compared to comparative combinations where same inorganic UV filters are dispersed in C12-15 alkyl benzoate or propylene glycol diheptanoate.

Combinations according to the invention present a very good wetting performance, meaning the combinations according to the invention can be easily spread on a surface.

### 2.3 Shear-thinning property

Dynamic viscosities of each combination according to the invention were measured using Rheometer MCR302, 24 hours after their preparation.

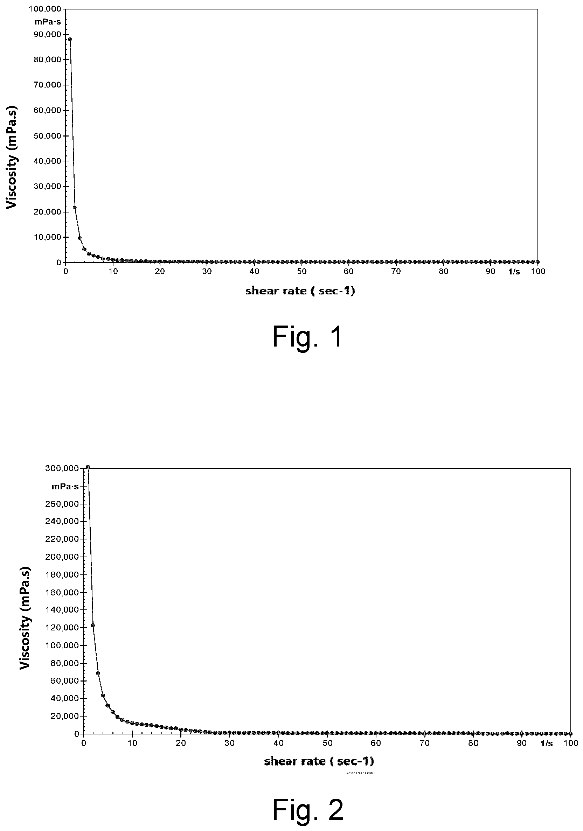

In Fig. 1 and Fig. 2, it can be observed that the viscosity of the combinations decreases when the shear rate increases. Combinations according to the invention are shear-thinning. It means that the inorganic UV filter particles contained in the combination according to the invention can be spread easily on the skin.

### Example 3: Cosmetic products

### 3.1 Mineral soothing sunscreen spray

Chemicals and their quantities are described in Table 2 below.

These oil-in-water mineral soothing sunscreen sprays were prepared according to the following steps:
Phase A was prepared by introducing the corresponding ingredients into the main beaker.

Phase A was then heated to 75°C until homogeneous.

Phase B was prepared by introducing the corresponding ingredients into a separate beaker under stirring at 800 rpm until homogeneous. Phase B was then heated to 75°C.

Phase B was added to phase A and homogenized using an Ultra-Turrax at 8000 rpm for 10 minutes.

Inorganic UV filters of phase C were then added to the mixture of phases A and B at around 40°C under stirring at 700-800 rpm.

Phase D was prepared by mixing corresponding ingredients in a separate beaker and heated at 40°C. Phase D was then added to mixture of phases A, B and C.

The pH was then controlled and adjusted if needed to 4-5 by adding citric acid (20% sol).

**Table 2: Mineral soothing sunscreen spray 1 according to the invention and comparative soothing sunscreen sprays 11 and 12**

| | INCI names | Function | Sunscreen spray 1 (%w/w) | Comparative sunscreen spray 11 (%w/w) | Comparative sunscreen spray 12 (%w/w) |
|---|---|---|---|---|---|
| A | Aqua | solvent | 67.1 | 67.1 | 67.1 |
| | Carbomer | thickening and/or gelling agent | 0.2 | 0.2 | 0.2 |
| | Allantoin | active ingredient | 0.5 | 0.5 | 0.5 |
| B | Glyceryl stearate and PEG-100 stearate | emulsifier | 3 | 3 | 3 |
| | Diisoamyl malate | emollient | 10 | | |
| | C12-15 alkyl benzoate | emollient | | 10 | |
| | Propylene glycol diheptanoate | emollient | | | 10 |
| C | Titanium dioxide | UV filter | 10 | 10 | 10 |
| | Zinc oxide | UV filter | 8 | 8 | 8 |
| D | Phenoxyethanol | preservative | 0.8 | 0.8 | 0.8 |
| | Perfume | odor masking | 0.3 | 0.3 | 0.3 |
| | Tocopherol acetate | active ingredient | 0.1 | 0.1 | 0.1 |
| E | Citric acid | pH modifier | appropriate amount | appropriate amount | appropriate amount |

### 3.2 Mineral sunscreen stick

Chemicals and their quantities are described in Table 3 below.

These mineral sunscreen sticks were prepared according to the following steps:
Phase A was prepared by introducing the corresponding ingredients into the main beaker.

Phase A was then heated to 85°C until melted, then it was cooled down to 50°C.

Phase B was prepared by introducing the corresponding ingredients in a separate beaker under stirring at 800 rpm until homogeneous. Phase B was then heated to 50°C.

Phase B was added to phase A under stirring at 700 rpm.

Phase C was prepared by mixing the corresponding ingredients.

Phase C was then added to the mixture of phases A and B under stirring at 700 rpm, until homogeneous.

**Table 3: Mineral sunscreen stick according to the invention and comparative mineral sunscreen stick**

| | | | | | |
|---|---|---|---|---|---|
| | INCI names | Function | Sunscreen stick 1 (%w/w) | Comparative sunscreen | Comparative sunscreen |

| | | | | stick 11 (%w/w) | stick 12 (%w/w) |
|---|---|---|---|---|---|
| A | Isohexatriocontentol, dilinoleyl alcohol, glycerol monocaprylate, ethyl cellulose | thickening and/or gelling agent | 36.6 | 36.6 | 36.6 |
| | Dibutyl ethylhexanoyl glutamide (and) dibutyl lauroyl glutamide (and) octyldodecanol | thickening and/or gelling agent | 22 | 22 | 22 |
| | Polyglyceryl-3 diisostearate | stick stabilizer | 3 | 3 | 3 |
| | Sunflower (Helianthus) seed oil | emollient | 10 | 10 | 10 |
| B | Diisoamyl malate | emollient | 10 | | |
| | C12-15 alkyl benzoate | emollient | | 10 | |
| | Propylene glycol diheptanoate | emollient | | | 10 |
| | Titanium dioxide | UV filter | 10 | 10 | 10 |
| | Zinc oxide | UV filter | 8 | 8 | 8 |
| C | Perfume | odor masking | 0.3 | 0.3 | 0.3 |
| | Tocopherol acetate | active ingredient | 0.1 | 0.1 | 0.1 |

### 3.3 SPF of cosmetic products

The SPF estimated by the BASF's Sunscreen Simulator, for the mixture of inorganic UV filters is of 38.30.

The SPF value of the cosmetic products prepared in Examples 3.1 and 3.2, were assed in-vitro.

Results are gathered in Table 4 and 5 below.

**Table 4: SPF of the oil-in-water mineral soothing sunscreen spray according to the invention and of the comparative oil-in-water soothing sunscreen sprays**

| | SPF |
|---|---|
| Sunscreen spray 1 | 38.33 |
| Comparative sunscreen spray 11 | 4.08 |
| Comparative sunscreen spray 12 | 5.50 |

The SPF of the sunscreen spray comprising diisoamyl malate as emollient and dispersing agent is much higher than the comparative sunscreen sprays.

Moreover, since the sunscreen spray according to the invention has a similar SPF (38.33), to the estimated SPF of inorganic filters used (38.30), it can be concluded that diisoamyl malate has a SPF boosting effect.

**Table 5: SPF of the mineral sunscreen stick according to the invention and of the comparative mineral sunscreen sticks**

| | SPF |
|---|---|
| Sunscreen stick 1 | 57.17 |
| Comparative sunscreen stick 11 | 26.56 |
| Comparative sunscreen stick 12 | 28.85 |

The mineral sunscreen stick comprising diisoamyl malate as emollient and dispersing agent, presents a very high SPF (more than 50).

In this formulation, the SPF is significantly higher that the estimated SPF. Diisoamyl malate can thus be considered as a SPF booster.

### 3.4 Whitening effect

To evaluate the whitening effect of the mineral sunscreen sticks on skin, measurements of melanin index and erythema index were performed using a Mexameter MX 18.

Sunscreen sticks prepared on Example 3.2 were applied on skin according to the method described below.

Calibration was performed with the Calibration Cap= 231-251 (reference values for melanin), 0-5 (Erythema/redness).

The skin areas were cleaned with an alcohol swab.

The non-treated skin was recorded to serve as a control and compare the color of the naked skin with the skin treated with each prepared sunscreen stick.

The sunscreen sticks were each applied on a cleaned part of the skin by applying each stick 3 strokes upward and 3 strokes downward.

The skin was allowed to dry at 25°C for 2 minutes.

Measurements were then taken.

Values of melanin index and of erythema index are given in Table 6 below:

**Table 6: Melanin index and erythema index of a skin treated with different sunscreen sticks**

| | Average melanin index | Average erythema index |
|---|---|---|
| Calibration | 243.67 | 0.00 |
| Non-treated Skin (Control) | 466.67 | 232.67 |
| Stick 1 according to the invention | 315.00 | 215.00 |
| Comparative stick 11 | 290.67 | 204.33 |
| Comparative stick 12 | 315.33 | 232.00 |

Average melanin index ranges from scale of 0-999 with increasing pigmentation. In this test, the skin of panel is highly pigmented at 466.67.

It can be observed that there is less difference between values of the average melanin index obtained without (control) and with the sunscreen stick according to the invention (466.67-315.00=151.67), than the difference between values of the average melanin index obtained without and with the comparative sunscreen stick (466.67-290.67= 176).

Consequently, there is less whitening effect of the skin with the sunscreen stick according to the invention comprising diisoamyl malate than with the comparative sunscreen sticks. It can be concluded that diisoamyl malate makes the UV filters well dispersed within the stick and so well-spread on the skin.

The index of color correction were then calculated based on melanin index (pigment) and erythema index (redness). It is the ratio (average melanin index of treated skin / average melanin index of non-treated skin) / (average erythema index of treated skin / average erythema index of non-treated skin).

Values of index of color correction are given in Table 7 below:

**Table 7: Index of color correction of different sunscreen sticks**

| | Index of color correction |
|---|---|
| Non-treated skin (control) | 1.00 |
| Sunscreen stick 1 | 0.73 |
| Comparative sunscreen stick 11 | 0.71 |
| Comparative sunscreen stick 12 | 0.73 |

The closest the value to the value of non-treated skin, the best it is.

The sunscreen stick according to the invention presents the closest value (0.73). It can be concluded that the sunscreen stick according to the invention is the stick with the most reduced whitening effect while being efficient against redness.

## Claims

1. Use of diisoamyl malate as a dispersing agent of titanium dioxide and/or zinc oxide.

2. Use according to claim 1, wherein the titanium dioxide and/or zinc oxide present(s) a particle size larger than 100 nm.

3. Combination consisting of diisoamyl malate and titanium dioxide and/or zinc oxide.

4. Process for preparing a combination according to claim 3, by mixing diisoamyl malate with titanium dioxide and/or zinc oxide.

5. Composition comprising:
- titanium dioxide and/or zinc oxide having a particle size larger than 100 nm; and
- diisoamyl malate.

6. Composition according to claim 5, wherein the quantity of diisoamyl malate is of at least 8% by weight based on the weight of the composition.

7. Composition according to claim 5 or 6, further comprising a thickening and/or gelling agent.

8. Composition according to any of claims 5 to 7, wherein the composition is free of water.

9. Composition according to any of claims 5 to 7, wherein the composition is in the form of an oil-in-water emulsion.

10. Process of preparation of a composition according to any of claims 5 to 9, comprising a step of dispersing titanium dioxide and/or zinc oxide in diisoamyl malate.

11. Use of the composition according to any of claims 5 to 9, as a cosmetic product.
